# EUROPÄISCHE PATENTSCHRIFT

(11) **EP 3 624 709 B1**
(45) Veröffentlichungstag und Bekanntmachung des Hinweises auf die Patenterteilung: **29.09.2021**
(21) Anmeldenummer: 18726368.6
(22) Anmeldetag: 11.05.2018
(51) Int. Cl.: A61B 17/22

(54) **STUHLIMPAKTIERUNG-ENTFERNUNGSVORRICHTUNG**
FECAL IMPACTION REMOVAL DEVICE
DISPOSITIF D'ÉLIMINATION DES SELLES DURES

(30) Priorität: 16.05.2017 DE 102017208203
(43) Veröffentlichungstag der Anmeldung: 25.03.2020
(73) Patentinhaber: Schauer, Georg, 5020 Salzburg (AT)
(72) Erfinder: Schauer, Georg, 5020 Salzburg (AT)
(74) Vertreter: Stolmár & Partner Patentanwälte PartG mbB
(86) Internationale Anmeldenummer: PCT/EP2018/062215
(87) Internationale Veröffentlichungsnummer: WO 2018/210694

(56) Entgegenhaltungen:
- CN-U- 203 263 450
- JP-U- 3 144 761
- US-A- 3 316 912
- US-A- 5 000 750
- US-A1- 2015 351 784
- US-A1- 2017 087 284

## Beschreibung

Die Erfindung betrifft eine Stuhlimpaktierung-Entfernungsvorrichtung zur Entfernung von Stuhlimpaktierung, vorzugsweise bei älteren Patienten.

Eine, meist altersbedingte Schwächung der Beckenbodenmuskulatur kann Stauungen des Stuhls im Endbereich des Mastdarms verursachen. Dabei entsteht eine meist kugelförmige Stuhlimpaktierung, die für Patienten extrem schmerzhaft und sogar lebensgefährlich wird. Es ist daher erforderlich, dass diese Stuhlimpaktierung mechanisch entfernt wird. Außer der Verwendung der Zeige- und/oder Mittelfinger des behandelnden Chirurgen oder Pflegers werden auch herkömmliche Esslöffel zur mechanischen Unterstützung der Zerlegung einer Stuhlimpaktierung verwendet.

Nachteilig bei dieser Unterstützung durch Esslöffel ist die dabei auftretende Verletzungsgefahr von Gewebe und Organen im Umfeld von Schließmuskulatur und/oder des Endbereichs des Mastdarms bis hin zur Prostata.

In US2015351784 wird ein Dekompressionsinstrument offenbart, mit dem Stuhl eines Patienten mit langjähriger chronischer Verstopfung entfernt werden kann. Das Instrument ist so konfiguriert, dass es über den Anus in den Magen-Darm-Trakt gelangt, um den betroffenen Stuhl zu entfernen. Der entfernte Stuhl kann vorübergehend in einem Auffangbeutel mit einem Rand mit Klebstoff gesammelt werden, um ihn während eines Dekompressionsvorgangs am Patienten zu befestigen.

Die vorliegende Erfindung hat die Aufgabe, eine Stuhlimpaktierung-Entfernungsvorrichtung zur Entfernung von Stuhlimpaktierung zu schaffen, bei der eine gezielte Entfernung von Teilen einer Stuhlimpaktierung einerseits mechanisch leichter durchführbar ist und andererseits eine Verletzungsgefahr reduziert oder sogar vermeidbar wird.

Die Aufgabe der Erfindung wird durch die im Anspruch 1 angegebenen Merkmale gelöst. Da der Löffelrand mindestens auf einer der beiden Seiten vom Löffel-Innenbereich über die Wandstärke nach außen eine nach oben abgerundete oder nach unten eingedellte

Randfläche aufweist, die nach oben abgerundete Randfläche im Randbereich zum umgebenden Gewebe besonders gewebeschonend und bei einem nach unten eingedellten Bereich eine sanfte Schnittkante bildet. Bei der Verwendung von zwei verschiedenen Randflächen-Formen kann durch Rechts- oder Links-Drehung ein sanfter oder schneidender Einsatz gesteuert werden. Die weiteren Vorteile der Erfindung werden durch die in den Unteransprüchen angegebenen Merkmale gelöst.

Ein weiterer Vorteil der Erfindung besteht darin, dass die Einführ- und Ausschab-Kräfte feiner und mechanisch stabiler steuerbar sind, als bei Verwendung von herkömmlichen Vorrichtungen oder Mittel. Der konusförmige Kopfbereich des Löffelbereichs weist vorzugsweise eine dickere Wandstärke als der Rest des Löffelbereichs, insbesondere als ein seitlicher Seitenwangenabschnitt des Löffelbereichs, auf. Im konusförmigen Kopfbereich weist der Löffelbereich beispielsweise eine um mehr als 50%, vorzugsweise eine um mehr als 100% dickere Wandstärke als im Seitenwangenabschnitt auf.

Ein weiterer Vorteil der Erfindung besteht darin, dass eine Entleerungshilfe vorgesehen werden kann, mit der nach jedem Ausschab-Vorgang die Entfernung eines Teils der Stuhlimpaktierung schneller und damit geruchsverringernd durchführbar ist.

Ein weiterer Vorteil der Erfindung besteht darin, dass eine Spülleitung vorgesehen werden kann, mit der vor und/oder nach jedem Ausschab-Vorgang die Entfernung eines Teils der Stuhlimpaktierung schneller und weniger anhaftend durchführbar ist.

Ein weiterer Vorteil der Erfindung besteht darin, dass ein Leitkanal, beispielsweise in Form der Spülleitung, vorgesehen werden kann, in den eine Lichtfaserleitung und/oder ein endoskopisches Werkzeug einführbar ist, womit die Konsistenz eines Teils der Stuhlimpaktierung vor und/oder nach jedem Ausschab-Vorgang und/oder der den Kopf der Vorrichtung umgebenden Bereich beobachtet und/oder untersucht werden kann.

Ein Vorteil der Erfindung wird durch einen Seitenwangenabschnitt zur Erhöhung des Löffelschalenbereichs erzielt, wodurch ein Fassungsvolumen des Löffelbereichs vergrößert wird. Durch eine einstückige Ausbildung des Löffelschalenbereichs mit dem Kopfbereich wird eine höhere Stabilität bei den auf den Löffelbereich einwirkenden Kräften und bei der Sterilisierung erreicht.

Ein weiterer Vorteil der Erfindung wird dadurch erreicht, dass der Griffbereich eine, vorzugsweise umlaufende, Griff-Mulde hinter dem an den Löffelschalenbereich anschließenden Löffelhals und einen daran anschließenden umlaufenden Griff-Verbreiterungsbereich und einen daran anschließenden, konisch auslaufenden Griff-Endkappen-Bereich aufweist. Dadurch lassen sich äußerlich eine sichere und feine Griffnutzung und eine räumliche Nutzung in einem Griff-Innenbereich realisieren.

Ein weiterer Vorteil der Erfindung wird dadurch erreicht, dass der Löffelrand, mindestens auf einer der beiden Seiten, eine vom Löffel-Innenbereich über die Wandstärke nach außen ansteigende Randfläche aufweist, sodass eine Schnittkante für extrem verhärteten Kot ausgestaltet ist.

Ein weiterer Vorteil der Erfindung wird dadurch erreicht, dass der konusförmige Kopfbereich, der eine sanfte Einführung in den Mastdarm-Eingang erlaubt, auf der Innenseite des Löffelschalenbereichs eine Konus-Rückseite mit einer ebenen, vorzugsweise zum oberen Löffelschalenbereich und Seitenwänden geneigt ist, wobei die Materialstärke im unteren Bereich des Kopfbereichs breiter als im oberen Bereich des Kopfbereichs ist, sodass eine leichtere Reinigung des Löffelschalenbereichs zusammen mit der Bodenseite, den Seitenwänden und der Löffelhals-Fläche möglich ist.

Ein weiterer Vorteil der Erfindung wird dadurch erreicht, dass im Innenraum des Griffbereichs mindestens eine Durchgangsöffnung vorgesehen ist, durch die mindestens eine Zusatzvorrichtung zum Innenbereich des Löffelbereichs eingesetzt oder eingeführt werden kann, wodurch z.B. ein zusätzlicher Zugang zum Löffelschalenbereich aus mehreren Gründen ermöglicht wird.

Ein weiterer Vorteil der Erfindung wird dadurch erreicht, dass in einer zwischen Löffelbereich und Löffelhals bestehende Zwischen-Wandung eine Aussparung vorgesehen ist, in die ein Stößel-Kopf der Stuhlimpaktierung-Entfernungsvorrichtung formbündig einsetzbar ist, der aus der Aussparung heraus in den Löffelschalenbereich bzw. einen Löffelschalen-Innbereich und damit beim Vorschieben des darin befindlichen Kots vollständig oder mindestens größtenteils ausgeschoben werden kann.

Ein weiterer Vorteil der Erfindung wird dadurch erreicht, dass der Stößel-Kopf mittels einer durch die im Griffbereich ausgebildeten Durchgangsöffnung mittels einer Zug- und Druckstange mit einem, im Bereich des Griff-Endkappen-Bereichs angeordneten Betätigungsgriff, in Richtung der Konus-Rückseite vorschoben und in die Ausgangsposition zurückgezogen werden kann.

Ein weiterer Vorteil der Erfindung wird dadurch erreicht, dass in dem Löffelbereich und/oder Kopfbereich eine Auslassöffnung von einem Leitkanal vorgesehen ist, aus der ein Fluid ausströmen kann. Das Fluid kann ein vorgewärmtes Wasser sein, das bei Bedarf mit z.B. einem Silicon-Öl versetzt die Oberflächen des Löffelbereichs gleitfähiger und damit für Kot weniger anhaftbar macht. Durch diesen oder einen weiteren Leitkanal im Griff und im Löffelbereich kann zudem ein Licht- und /oder Optik-Kopf und/oder ein endoskopisches Instrument herausgeschoben werden. Dadurch kann sowohl eine Beleuchtung als auch eine Manipulation im inneren und/oder äußeren Umfeld des Löffelbereichs erreicht werden.

Ein weiterer Vorteil der Erfindung wird dadurch erreicht, dass mit einer aus dem Griffbereich im Anschluss an den Durchgangsbereich herausführenden Anschluss-Vorrichtung über die funktionelle Verbindung des an der Auslassöffnung vorgesehenen Funktionskopfes ein Fluidbehälter anschließbar ist. Operativ wird durch die Anschluss-Vorrichtung im Griff-Endkappen-Bereich eine wenig störende Handhabung ermöglicht.

Ein weiterer Vorteil der Erfindung wird dadurch erreicht, dass die Anschlussvorrichtung mit einem mit Reinigungs-Fluid gefüllten Griff-Ball verbunden ist, sofern der Druck für die Fluideinbringung manuell aufgebracht wird. Im Falle einer druckbeaufschlagten Fluid-Versorgung kann der Durchfluss durch einen den Durchfluss-Querschnitt steuernden Griffring erreicht werden. Auch die Steuerung von Lichtquellen und endoskopischen Geräten kann über den Griffring gesteuert werden, wenn zusätzliche Signal-Leitungen durch den Leitkanal zum Griffring geführt werden.

Nachfolgend wird die Erfindung anhand der beigefügten Zeichnungen näher beschrieben. Darin zeigt:
- Figur 1:: Eine seitliche Schnittansicht der erfindungsgemäßen Vorrichtung
- Figur 2:: Eine geschnittene Draufsicht auf die erfindungsgemäße Vorrichtung

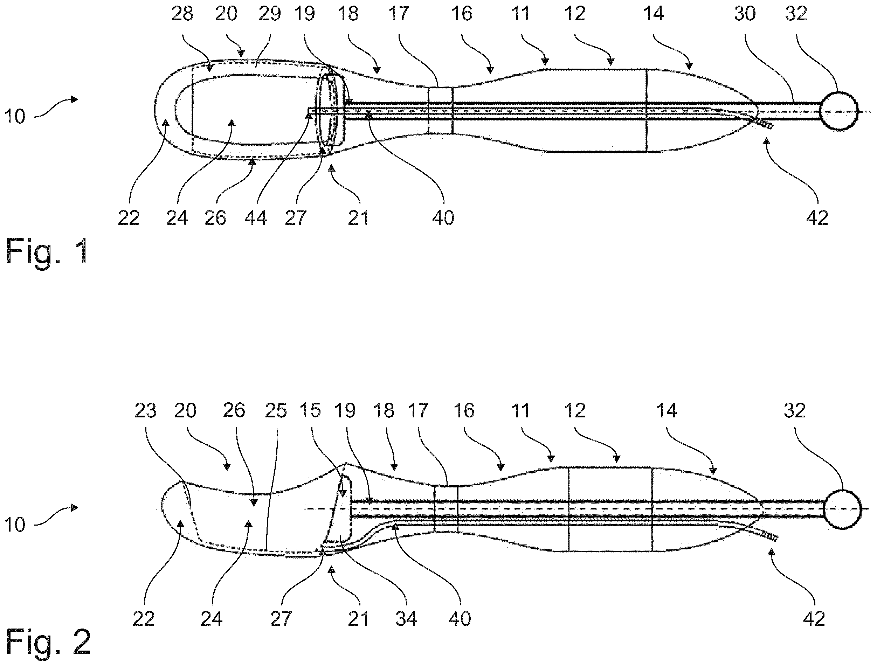

In Figur 1 weist die erfindungsgemäße Stuhlimpaktierung-Entfernungsvorrichtung 10 einen Griffbereich 11 auf, der einen Griff-Verbreiterungsbereich 12, einen Griff-Endkappen-Bereich 14 und einen Griff-Konus 16 aufweist. Zwischen Griff-Verbreiterungsbereich 12 und Griff-Konus 16 oder im Anschluss an den Griff-Konus 16 ist ein Griffring 17 eingesetzt und bildet zusammen mit dem Griff-Verbreiterungsbereich 12, dem Griff-Konus 16, dem Griffring 17 und einem Löffelhals 21 eine Griff-Mulde 18. Durch den Griffbereich 11 führt vorzugsweise in einem Griff-Achsenbereich eine Durchgangsöffnung 19.

An den Griffbereich 11 schließt sich ein Löffelbereich 20 mit dem Löffelhals 21 an. Ein dreidimensional konusförmiger, nach vorne gerichteter, abgerundeter Kopfbereich 22 bildet den vorderen Abschluss des Löffelbereichs 20. Der Kopfbereich 22 weist eine Konus-Rückseite 23 auf, die zusammen mit einem Bodenabschnitt 25, einer Zwischen-Wandung 27 am Löffelhals 21 und einem Seitenwangenabschnitt 26 bzw. einem Löffelrand 28 insgesamt einen Löffelschalenbereich 24 bilden. Vorzugsweise weist der konusförmige Kopfbereich mindestens eine, zum Innenbereich des Löffelschalenbereichs 24 gerichtete Konus-Rückseite 23 auf, die eine vorzugsweise ebene Fläche aufweist, die vom Bodenabschnitt 25 zu einem oberen Seitenwangenabschnitt 26 bzw. zu einem Löffelrand 28 so geneigt ist, dass der untere Bereich des Kopfbereiches 22 breiter als der obere Bereich des Kopfbereiches 22 ist. Der Löffelrand 28 weist eine Randfläche 29 auf, die mindestens auf einer der beiden Seiten eine vom Löffelschalenbereich 24 über die Wandstärke des Löffelrands 28 nach oben außen ansteigende Randfläche 29 aufweist. Dadurch ergibt sich eine Schnittkante mit der eine besonders kompakte Stuhlimpaktierung leichter abschneidbar ist. Wenn der Löffelrand 28, mindestens auf einer der beiden Seiten, vom Löffel-Innenbereich über die Wandstärke nach außen eine nach oben oder unten abgerundete Randfläche 29 aufweist, dann können Teile der Stuhlimpaktierung gewebeschonend entfernt werden.

Der Griffbereich 11 weist eine in die sich von dem Löffelschalenbereich 24 zum hinteren Ende des Griff-Konus 16 erstreckende Durchgangsöffnung 19 einsetzbare Zug- und Druckstange 30 auf, die aus dem Griff-Konus 16 heraus in einem Betätigungsgriff 32 mündet.

In der Zwischenwandung 27 des Löffelschalenbereichs 24 ist eine Aussparung 15 vorgesehen, in die ein Stößel-Kopf 34, der an dem anderen Ende der Zug- und Druckstange 30 befestigt ist, form-und oberflächen-bündig eingesetzt ist. Mittels des Betätigungsgriffs 32 kann die Zug- und Druckstange 30 und der damit verbundene Stößel-Kopf 34 in den Hohlraum des Löffelschalenbereichs 24 hinein bis zur Konus-Rückseite 23 vorgeschoben und bis in die Aussparung 15 zurückgezogen werden. Um einen volumenmäßig optimalen Auswurf von Teilen der Stuhlimpaktierung aus dem Löffelschalenbereich 24 zu erreichen, ist die, dem Löffelschalenbereich 24 zugewandte Seite des Stößel-Kopfes 34, mit einer weitgehend die Zwischenwandung ausfüllenden Oberfläche versehen, sodass der verbleibende Randbereich von 1 bis 5 Millimeter für Stabilität und mindestens eine Auslass-Öffnung 44 ausreichend dimensioniert ist. Gleiches gilt für den Durchmesser des Bodenabschnitts 25.

Die Auslass-Öffnung 44 öffnet sich aus dem Leitkanal 40, die durch den Griffbereich 11 bis zu der hinteren Auslauföffnung führt. Für eine fluidführende Durchgangsleitung durch den Leitkanal ist ein Fluidzufluss vorzugsweise mit einer Anschlussvorrichtung 42 versehen, an welcher ein fluidhaltiger Behälter anschließbar ist. Vorzugsweise kann der fluidhaltige Behälter als ballähnlicher Kunststoff-Behälter ausgebildet sein, sodass darauf einwirkende Druckkräfte das darin enthaltene Fluid aus der Auslass-Öffnung 44 in den Innenbereich des Löffelschalenbereichs 24 oder auch bei Bedarf und entsprechender Leitungsführung an den Außenbereich des Löffelbereichs 20 in die Umgebung ausleitet. Das Fluid kann Wasser oder eine andere Reinigungslösung sein und auch Silikon-Öl oder dgl. enthalten, um die Oberflächen des Löffelbereichs 24 gegen stärkere Anhaftungen zu schützen. Natürlich kann auch ein anderer fluidhaltiger Behälter mit einem druckbeaufschlagten Fluid an die Anschlussvorrichtung 42 angeschlossen sein. Der Durchfluss kann dabei mit einem Griffring 17 dosiert geöffnet werden. Mit dem Griffring 17 können auch andere Signale für Beleuchtungs- und oder Optik-Einsätze oder Aktivitäten von endoskopischen Instrumenten und deren Signalleitungen oder Funk oder Magnet-Verbindungen, gesteuert werden.

Die Stuhlimpaktierung-Entfernungsvorrichtung zur Entfernung von Stuhlimpaktierung kann aus leicht zu desinfizierenden Material wie Edelstahl oder einem Einwegmaterial wie leicht recyclefähigem Kunststoff einstückig oder aus Kopfbereich und Griffbereich getrennt, aber zusammensetzbar oder zusammensteckbar hergestellt sein.

### Bezugszeichenliste

- 10: Handliche Vorrichtung
- 11: Griffbereich
- 12: Griff-Verbreiterungsbereich
- 14: Griff-Endkappen-Bereich
- 15: Aussparung
- 16: Griff-Konus
- 17: Griffring
- 18: Griff-Mulde
- 19: Durchgangsöffnung
- 20: Löffelbereich
- 21: Löffelhals
- 22: Kopfbereich
- 23: Konus-Rückseite
- 24: Löffelschalenbereich
- 25: Bodenabschnitt
- 26: Seitenwangenabschnitt
- 27: Zwischen-Wandung
- 28: Löffelrand
- 29: Randfläche
- 30: Zug- und Druckstange
- 32: Betätigungsgriff.
- 34: Stößel-Kopf
- 40: Leitkanal
- 42: Anschluss-Vorrichtung
- 44: Auslassöffnung

## Patentansprüche

1. Stuhlimpaktierung-Entfernungsvorrichtung (10) zur Entfernung von Stuhlimpaktierung, mit einem Griffbereich (11) und einem Löffelbereich (20), wobei der Löffelbereich (20) am vorderen Ende einen dreidimensional konusförmigen Kopfbereich (22) aufweist, an den sich in Richtung des Griffbereich (11) ein Löffelschalenbereich (24) anschließt;
**dadurch gekennzeichnet, dass**
der Löffelrand (28), mindestens auf einer der beiden Seiten, vom Innenbereich des Löffelschalenbereichs (24) eine über die Wandstärke des Seitenwangenabschnitts (26) nach außen eine, nach oben gerundete oder unten eingedellte Randfläche aufweist.

2. Stuhlimpaktierung-Entfernungsvorrichtung (10) nach Anspruch 1, **dadurch gekennzeichnet, dass** der Kopfbereich (22) zumindest abschnittsweise abgerundet ist.

3. Stuhlimpaktierung-Entfernungsvorrichtung (10) nach Anspruch 1, **dadurch gekennzeichnet, dass** der Löffelschalenbereich (24) einen Seitenwangenabschnitt (26) mit Seitenwangen aufweist, die, vorzugsweise einstückig, mit dem Löffelschalenbereich (24) und/oder dem Kopfbereich (22) ausgebildet sind.

4. Stuhlimpaktierung-Entfernungsvorrichtung (10) nach einem der vorhergehenden Ansprüche, **dadurch gekennzeichnet, dass** das Griffbereich (11) eine, vorzugsweise umlaufende, Griff-Mulde (18) nach dem Löffelschalenbereich (24) und/oder einen daran anschließenden, vorzugsweise umlaufenden, Griff-Verbreiterungsbereich (12) und/oder einen daran anschließenden, vorzugsweise konisch auslaufenden, Griff-Endkappen-Bereich (14 ) aufweist.

5. Stuhlimpaktierung-Entfernungsvorrichtung (10) nach einem der vorhergehenden Ansprüche, **dadurch gekennzeichnet, dass** ein Löffelrand (28) mindestens auf einer der beiden Seiten vom Innenbereich des Löffelschalenbereichs (24) eine über die Wandstärke nach außen ansteigende Randfläche (29) aufweist.

6. Stuhlimpaktierung-Entfernungsvorrichtung (10) nach einem der vorhergehenden Ansprüche, **dadurch gekennzeichnet, dass** der konusförmige Kopfbereich (22) mindestens eine, zu einem Innenbereich des Löffelschalenbereichs (24) gerichtete Konus-Rückseite (23), vorzugsweise in Form einer ebenen Fläche, aufweist, die vorzugsweise von einem Bodenabschnitt (25) des Löffelschalenbereichs (24) zu einem oberen Seitenwangenabschnitt (26) des Löffelschalenbereichs (24) so geneigt ist, dass der untere Bereich des Kopfbereiches (22) breiter als der obere Bereich des Kopfbereiches ist.

7. Stuhlimpaktierung-Entfernungsvorrichtung (10) nach einem der vorhergehenden Ansprüche, **dadurch gekennzeichnet, dass** in dem Griffbereich mindestens eine Durchgangsöffnung (19) vorgesehen ist, durch die mindestens eine Zusatzvorrichtung (30, 32, 34) zum Innenbereich des Löffelschalenbereichs (24) eingesetzt und/oder eingeführt werden kann.

8. Stuhlimpaktierung-Entfernungsvorrichtung (10) nach einem der vorhergehenden Ansprüche, **dadurch gekennzeichnet, dass**, in eine, zwischen dem Löffelschalenbereich (24) und einem Löffelhals (21) bestehende Zwischen-Wandung (27), eine Aussparung (15) vorgesehen ist, in die ein Stößel-Kopf (34) der Stuhlimpaktierung-Entfernungsvorrichtung (10) formbündig eingesetzt oder einsatzbar ist.

9. Stuhlimpaktierung-Entfernungsvorrichtung (10) nach Anspruch 8, **dadurch gekennzeichnet, dass** der Stößel-Kopf (34) mittels einer in der Durchgangsöffnung (19) geführten Zug- und Druckstange (30) in den Löffelbereich (24) bis zur Konus-Rückseite (23) vorschiebbar und in die Ausgangsposition rückziehbar gelagert ist, vorzugsweise mittels einem, im Bereich des Griff-Endkappen-Bereichs (14) angeordneten Betätigungsgriff (32), der bevorzugt zumindest abschnittsweise einen größeren Durchmesser als die Zug- und Druckstange (30) aufweist.

10. Stuhlimpaktierung-Entfernungsvorrichtung (10) nach einem der vorhergehenden Ansprüche, **dadurch gekennzeichnet, dass** in dem Löffelbereich und/oder Kopfbereich eine Auslassöffnung (44) eines Leitkanals (40) vorgesehen ist, aus der ein Fluid in den Löffelschalenbereich (24) ausströmen und/oder ein Licht- und /oder Optik-Kopf und/oder ein endoskopisches Instrument in den Löffelschalenbereich (24) herausgeschoben werden kann.

11. Stuhlimpaktierung-Entfernungsvorrichtung (10) nach Anspruch 10, **dadurch gekennzeichnet, dass** eine Anschluss-Vorrichtung (42) für eine funktionelle Verbindung der im Löffelhals (21) zum Löffelschalenbereich (24) hin gerichteten Auslassöffnung (44) durch den Leitkanal (40) im Griff-Endkappen-Bereich (14) vorgesehen ist.

12. Stuhlimpaktierung-Entfernungsvorrichtung (10) nach Anspruch 11, **dadurch gekennzeichnet, dass** die Anschlussvorrichtung (42) mit einem mit einem Reinigungs-Fluid gefüllten Griff-Ball verbunden ist.

## Claims

1. Fecal impaction removal device (10) for removing fecal impaction, comprising a handle region (11) and a scoop region (20), the scoop region (20) at the front end having a three-dimensionally cone-shaped head region (22), which is followed by a bowl-shaped region (24) in the direction of the handle region (11);
**characterized in that**
the scoop edge (28), at least on one of the two sides, from the inner region of the bowl-shaped region (24) has an edge surface that is rounded upwards or indented at the bottom over the wall thickness of the side cheek portion (26) to the outside.

2. Fecal impaction removal device (10) according to claim 1, **characterized in that** the head region (22) is at least partially rounded.

3. Fecal impaction removal device (10) according to claim 1, **characterized in that** the bowl-shaped region (24) has a side cheek portion (26) with side cheeks, which are preferably integrally formed with the bowl-shaped region (24) and/or the head region (22).

4. Fecal impaction removal device (10) according to one of the preceding claims, **characterized in that** the handle region (11) has a grip recess (18), preferably circumferential, after the bowl-shaped region (24) and/or has an adjoining, preferably circumferential, handle widening region (12) and/or an adjoining, preferably conically tapering, handle end cap region (14).

5. Fecal impaction removal device (10) according to any one of the preceding claims, **characterized in that** a scoop edge (28) has an edge surface (29) rising outward over the wall thickness at least on one of the two sides of the inner region of the bowl-shaped region (24).

6. Fecal impaction removal device (10) according to one of the preceding claims, **characterized in that** the cone-shaped head region (22) has at least one conical rear side (23) directed towards an inner region of the bowl-shaped region (24), preferably in the form of a flat surface, which is preferably inclined from a bottom portion (25) of the bowl-shaped region (24) to an upper side cheek portion (26) of the bowl-shaped region (24) so that the lower region of the head region (22) is wider than the upper region of the head region.

7. Fecal impaction removal device (10) according to any one of the preceding claims, **characterized in that** at least one through opening (19) is provided in the handle region through which at least one additional device (30, 32, 34) is inserted to the interior of the bowl-shaped region (24) and/or can be introduced.

8. Fecal impaction removal device (10) according to one of the preceding claims, **characterized in that** a recess (15) is provided in an intermediate wall (27) existing between the bowl-shaped region (24) and a scoop neck (21), into which a plunger head (34) of the fecal impaction removal device (10) is inserted or can be inserted flush with the shape.

9. Fecal impaction removal device (10) according to claim 8, **characterized in that** the plunger head (34) by means of a pull and push rod (30) guided in the through opening (19) into the bowl-shaped region (24) up to the conical rear side (23) can be advanced and retracted into the starting position, preferably by means of an actuating handle (32) which is arranged in the region of the handle end cap region (14) and preferably at least partially has a larger diameter than the pull and push rod (30).

10. Fecal impaction removal device (10) according to any one of the preceding claims, **characterized in that** an outlet opening (44) of a guide channel (40) is provided in the bowl region and/or head region, from which a fluid flows into the bowl-shaped region (24) and/or a light and/or optical head and/or an endoscopic instrument can be pushed out into the bowl-shaped region (24).

11. Fecal impaction removal device (10) according to claim 10, **characterized in that** a connection device (42) for a functional connection of the outlet opening (44) directed in the scoop neck (21) towards the bowl-shaped region (24) through the guide channel (40) is provided in the handle end cap region (14).

12. Fecal impaction removal device (10) according to claim 11, **characterized in that** the connection device (42) is connected to a grip ball filled with a cleaning fluid.

## Revendications

1. Dispositif d'élimination des selles dures (10) pour éliminer les selles dures, avec une zone de préhension (11) et une zone de cuillère (20), la zone de cuillère (20) ayant une zone de tête conique tridimensionnelle (22) à l'extrémité avant, suivie en direction de la zone de préhension (11) par une zone de coque de cuillère (24) ;
**caractérisé en ce que**
le bord de cuillère (28), au moins sur l'un des deux côtés, depuis la zone intérieure de la zone de coque de cuillère (24) présente une surface de bord qui est arrondie vers le haut ou indentée en bas sur l'épaisseur de paroi de la section de joue latérale (26) vers l'extérieur.

2. Dispositif d'élimination des selles dures (10) selon la revendication 1, **caractérisé en ce que** la zone de tête (22) est arrondie au moins par sections.

3. Dispositif d'élimination des selles dures (10) selon la revendication 1, **caractérisé en ce que** la zone de coque de cuillère (24) présente une section de joue latérale (26) avec des joues latérales qui sont de préférence formées en un seul tenant avec la zone de coque de cuillère (24) et/ou la zone de tête (22) .

4. Dispositif d'élimination des selles dures (10) selon l'une des revendications précédentes, **caractérisé en ce que** la zone de préhension (11) présente un creux de préhension (18) de préférence circonférentiel après la zone de coque de cuillère (24) et/ou une zone adjacente à celle-ci, de préférence circonférentielle, d'élargissement de préhension (12) et/ou une zone de capuchon d'extrémité de préhension (14) adjacente à celle-ci, de préférence effilée de manière conique.

5. Dispositif d'élimination des selles dures (10) selon l'une des revendications précédentes, **caractérisé en ce qu'**un bord de cuillère (28) présente une surface de bord (29) s'élevant vers l'extérieur sur l'épaisseur de paroi sur au moins l'un des deux côtés de la zone intérieure de la zone de coque de cuillère (24).

6. Dispositif d'élimination des selles dures (10) selon l'une des revendications précédentes, **caractérisé en ce que** la zone de tête conique (22) présente au moins un côté arrière du cône (23) tourné vers une zone intérieure de la zone de coque de cuillère (24), de préférence sous la forme d'une surface plane, qui est de préférence inclinée à partir d'une section de fond (25) de la zone de coque de cuillère (24) vers une section de joue latérale supérieure (26) de la zone de coque de cuillère (24) de sorte que la zone inférieure de la zone de tête (22) est plus large que la zone supérieure de la zone de tête.

7. Dispositif d'élimination des selles dures (10) selon l'une des revendications précédentes, **caractérisé en ce qu'**au moins une ouverture traversante (19) est prévue dans la zone de préhension, à travers laquelle au moins un dispositif supplémentaire (30, 32, 34) peut être inséré et/ou introduit dans la zone intérieure de la zone de coque de cuillère (24).

8. Dispositif d'élimination des selles dures (10) selon l'une des revendications précédentes, **caractérisé en ce qu'**un évidement (15) est prévu dans une paroi intermédiaire (27) existant entre la zone de coque de cuillère (24) et un col de cuillère (21), dans lequel une tête de poussoir (34) du dispositif d'élimination des selles dures (10) est insérée ou peut être insérée à niveau.

9. Dispositif d'élimination des selles dures (10) selon la revendication 8, **caractérisé en ce que** la tête de poussoir (34) au moyen d'une tige de traction et de poussée (30) à travers l'ouverture traversante (19) est montée coulissante vers l'avant dans la zone de cuillère (24) jusqu'au côté arrière du cône (23) et de façon rétractable dans la position de départ, de préférence au moyen d'une poignée de manœuvre (32) qui est disposée dans la zone de capuchon d'extrémité de préhension (14) et qui a de préférence au moins par sections un diamètre plus grand que la tige de traction et de poussée (30).

10. Dispositif d'élimination des selles dures (10) selon l'une des revendications précédentes, **caractérisé en ce qu'**une ouverture de sortie (44) d'un canal de guidage (40) est prévue dans la zone de cuillère et/ou la zone de tête, à partir de laquelle un fluide peut s'écouler dans la zone de coque de cuillère (24) et/ou une tête lumineuse et/ou optique et/ou un instrument endoscopique peut être expulsé(e) dans la zone de coque de cuillère (24).

11. Dispositif d'élimination des selles dures (10) selon la revendication 10, **caractérisé en ce qu'**un dispositif de connexion (42) est prévu pour une connexion fonctionnelle de l'ouverture de sortie (44) qui dans le col de cuillère (21) est tournée vers la zone de coque de cuillère (24) à travers le canal de guidage (40) dans la zone de capuchon d'extrémité de préhension (14).

12. Dispositif d'élimination des selles dures (10) selon la revendication 11, **caractérisé en ce que** le dispositif de connexion (42) est relié à une bille de préhension remplie d'un fluide de nettoyage.
